# EUROPÄISCHE PATENTANMELDUNG

(11) **EP 1 818 050 A1**
(43) Veröffentlichungstag der Anmeldung: **15.08.2007**
(21) Anmeldenummer: 06002725.7
(22) Anmeldetag: 10.02.2006
(51) Int. Cl.: A61K 9/48, A61K 9/28, A61K 31/505, A61K 31/22, A61K 31/404, A61K 31/40

(54) **Stabile pharmazeutische Zusammensetzungen umfassend einen HMG-CoA-Reduktase Inhibitor**

(71) Anmelder: STADA ARZNEIMITTEL AG, 61118 Bad Vilbel (DE)
(72) Erfinder: Renz, Jessica, 60594 Frankfurt (DE); Rillmann, Thomas, Dr., 76756 Bellheim (DE)
(74) Vertreter: Hamm, Volker

(57) **Zusammenfassung**

Die Erfindung betrifft neuartige Zusammensetzungen von HMG-CoA-Reduktase Inhibitoren, die eine hervorragende Stabilität aufweisen und ohne Zusatz einer alkalisierenden Verbindung formuliert sind.

## Beschreibung

Die Erfindung betrifft neue galenische Formulierungen von Dihydroxyheptansäuren und Dihydroxyheptensäuren sowie deren Salzen, insbesondere von pharmazeutischen Wirkstoffen aus der Klasse der HMG-CoA-Reduktase Inhibitoren (Statine), die stabil sind, obwohl sie einen pH-Wert von weniger als 8 aufweisen und obwohl bei ihrer Formulierung kein alkalischer Hilfsstoff eingesetzt wird, der in der Lage wäre, den pH-Wert auf einen entsprechenden Wert oberhalb von 8 zu erhöhen. Die vorliegende Erfindung betrifft zudem Verfahren zur Herstellung solcher Formulierungen.

Es wird allgemein angenommen, dass die Statine vom Typ der 3,5-Dihydroxyheptansäuren und 3,5-Dihydroxyheptensäuren, wie Fluvastatin, Pravastatin, Atorvastatin oder Rosuvastatin sowie deren Salze, in saurer Umgebung nicht stabil sind und dass deshalb galenische Formulierungen, die einen derartigen Wirkstoff enthalten, alkalisch reagierende Verbindungen enthalten müssen.

Die geschilderte Empfindlichkeit der HMG-CoA-Reduktase-Inhibitoren vom Dihydroxyheptan- und Dihydroxyheptensäuren-Typ allgemein und von Atorvastatin Calcium im speziellen wurde bereits in US 5,686,104 bzw. US 6,126,971 beschrieben. Um trotz der bei der Formulierung von Atorvastatin-haltigen Arzneimitteln beobachteten Probleme stabile pharmazeutische Darreichungsformen zu erhalten, wird in diesen Dokumenten ein Zusatz von Metallsalzen, die in wässriger Lösung alkalisch reagieren, vorgeschlagen.

Weitere Beispiele für solche stabilen Formulierungen mit alkalischen Hilfsstoffen für den HMG-CoA-Reduktase-Inhibitor Fluvastatin bzw. dessen Natriumsalz (Natrium Fluvastatin) wurden in DE 42 40 430 angegeben. In diesem Dokument und dem parallelen EP 0 547 000 wird ausgeführt, dass durch Verwendung eines alkalischen Mediums als Hilfsstoff, der den pH-Wert einer wässrigen Lösung oder Dispersion der pharmazeutischen Zubereitung auf wenigstens 8 einstellt, verbesserte Lagerfähigkeiten erhalten werden. Erst durch Verwendung des alkalischen Mediums werden gemäß DE 42 40 430 pharmazeutisch akzeptable Stabilitäten erhalten. Als mögliche alkalische Substanzen werden in DE 42 40 430 Carbonate, Bicarbonate, Phosphate und Hydroxide genannt.

EP 0 336 298 beschreibt für den HMG-CoA-Reduktase-Inhibitor Pravastatin, dass auch dieser Wirkstoff gegenüber niedrigen pH-Werten empfindlich ist und sich unter solchen Bedingungen in das korrespondierende Lakton und verschiedene andere Isomere umsetzt. Zur Lösung dieses Problems werden in EP 0 336 298 Formulierungen vorgeschlagen, in denen durch Zusatz einer basifizierenden Substanz (wie etwa Alkali- und Erdalkalimetalloxide und -hydroxide) der pH-Wert auf wenigstens 9 eingestellt wird.

WO 01/93860 führt schließlich aus, dass neben Pravastatin und Fluvastatin auch die anderen HMG-CoA-Reduktase-Inhibitoren vom 3,5-Dihydroxyheptan- bzw. 3,5-Dihydroxyheptensäure-Typ wie Atorvastatin, Rosuvastatin, Pitavastatin und Cerivastatin hochempfindlich gegenüber sauren Bedingungen sind, da es unter sauren Bedingungen zur Lactonisierung der 3,5-Dihydroxycarbonsäuren kommen könnte. Diese Problematik tritt insbesondere bei der Verwendung der Metallsalze dieser Statine auf. Das somit vom Stand der Technik eindeutig identifizierte Problem der Säurelabilität der Statine vom 3,5-Dihydroxyheptan- und 3,5-Dihydroxyheptensäure-Typ wird gemäß WO 01/93860 dadurch gelöst, dass die entsprechenden Statine mit puffernden oder basifizierenden Verbindungen co-kristallisiert oder gemeinsam ausgefällt werden. Auf diese Weise gelingt es, lagerstabile Zubereitungen z.B. von Pravastatin Natrium zu erhalten.

Im Stand der Technik bestand und besteht das Vorurteil, dass lagerstabile Formulierungen von HMG-CoA-Reduktase-Inhibitoren des 3,5-Dihydroxyheptan- und 3,5-Dihydroxyheptensäure-Typs nur durch Zusatz eines alkalischen Hilfsstoffs oder durch Co-Kristallisation bzw. gemeinsamer Ausfällung mit einer solchen Verbindung zu erhalten wären. Damit ist aber die Auswahl möglicher Hilfsstoffe beschränkt bzw. die Herstellung der entsprechenden Rezepturen aufwendig.

Es besteht somit ein großes Bedürfnis für Arzneimittel, die Statine vom 3,5-Dihydroxyheptan- bzw. 3,5-Dihydroxyheptensäure-Typ, insbesondere Fluvastatin, Atorvastatin oder Rosuvastatin sowie ähnliche Verbindungen und ihre Salze, als Wirkstoff enthalten, die eine hervorragende Stabilität kombiniert mit einer guten Bioverfügbarkeit haben und die nicht die Probleme und Nachteile einiger Arzneimittel des Standes der Technik zeigen. Die erfindungsgemäßen Formulierungen, die die im Stand der Technik beschriebenen Probleme und Bedürfnisse lösen, werden durch die Ansprüche näher charakterisiert.

Die vorliegende Erfindung beruht auf dem unerwartetem Befund, dass die Dihydroxy-Statine, die bisher für sehr säurelabil gehalten wurden, tatsächlich eine hervorragende Stabilität aufweisen, d.h. keinen Zersetzungs- bzw. Umlagerungsreaktionen unterliegen oder Verfärbungen zeigen, wenn sie ohne Zusatz einer alkalisierenden Verbindung, die in der Lage wäre, den pH-Wert einer wässerigen Lösung oder Dispersion der Formulierung auf einen pH-Wert von wenigstens 8 einzustellen, formuliert werden. Hierzu ist es keinesfalls notwendig, den Wirkstoff mit einer Base oder einer puffernden Substanz zu co-kristallisieren oder gemeinsam auszufällen.

Die Erfindung stellt somit stabile orale Arzneimittel zur Verfügung, die HMG-CoA-Reduktase Inhibitoren vom Dihydroxyheptansäure-Typ (Iₐ) bzw. vom 3,5-Dihydroxyheptensäure-Typ (I_{b}) 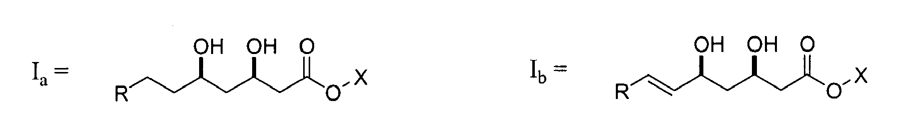 umfassen, worin R jeder organische Rest sein kann aber vorzugsweise aus 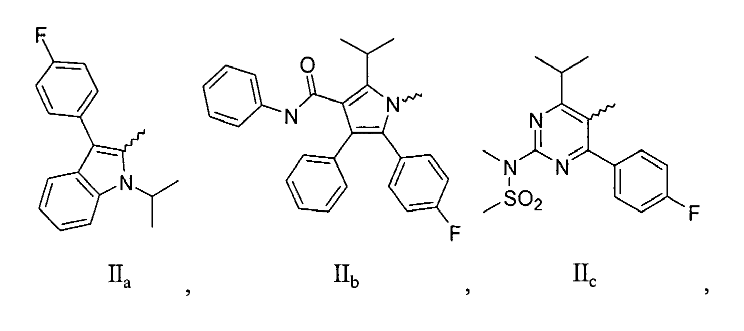 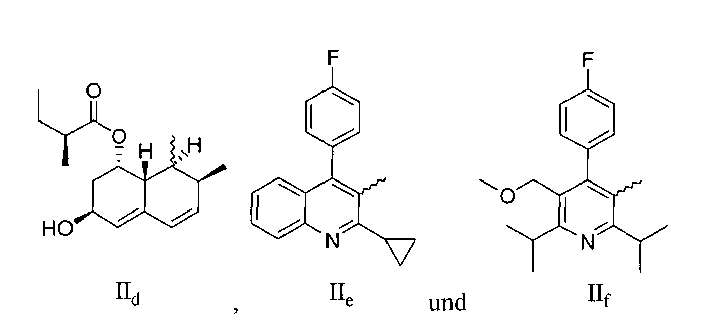 ausgewählt ist, und X aus der Gruppe Wasserstoff und Metall ausgewählt ist; insbesondere werden erfindungsgemäß R = IIₐ, II_{b}, II_{c} oder II_{d} und X = Na oder Ca_{½} bevorzugt.

Die erfindungsgemäßen pharmazeutischen Zusammensetzungen mit ausgezeichneter Lagerstabilität enthalten als pharmazeutischen Wirkstoff ein 3,5-Dihydroxy-Statin, insbesondere Fluvastatin (I_{b} mit R = IIₐ), Atorvastatin (Iₐ mit R = II_{b}), Rosuvastatin (I_{b} mit R = II_{c}), oder Pravastatin (Iₐ mit R = II_{d}), oder deren Salze, vorzugsweise X = Na oder X = Ca_{½}; besonders bevorzugt Fluvastatin Natrium (I_{b} mit R = IIₐ und X = Na), Atorvastatin Calcium (I_{b} mit R = II_{b} und X = Ca_{½}) und Rosuvastatin Calcium (Iₐ mit R = II_{c} und X = Ca_{½}); ganz besonders bevorzugt Fluvastatin Natrium (I_{b} mit R = IIₐ und X = Na). Dabei können die pharmazeutischen Mittel den aktiven Wirkstoff in jeder Konzentration enthalten, erfindungsgemäß werden 0,5-60 Gew.-% des Wirkstoffes relativ zum Gesamtgewicht der Darreichungsform bevorzugt.

Mögliche nicht alkalisierende Hilfsstoffe der erfindungsgemäßen Zusammensetzungen umfassen alle pharmazeutisch akzeptablen, nicht sauer reagierenden Substanzen, insbesondere mikrokristalline und mikrofeine Cellulose, Celluloseether, Lactose, mehrwertige Zucker, Polyacrylsäurederivate, Carrageene, Polyvinylpyrrolidon, Polyvinylpyrrolidon quervernetzt, Polyvinylalkohol, Polyvinylacetat, Magnesiumstearat, hochdisperse Kieselsäure (Siliciumdioxid) sowie Talcum. Bevorzugte Hilfsstoffe sind mikrofeine Cellulose, mit Polyvinylpyrrolidon und quervernetztem Polyvinylpyrrolidon vorbehandelte Lactose sowie Carrageene. Alle genannten Hilfsstoffe werden bevorzugt in kommerziell erhältlicher Qualität eingesetzt; besonders bevorzugt ohne alkalische Verunreinigungen.

Als mögliche Arzneimittelformen der erfindungsgemäßen Zusammensetzungen kommen insbesondere Tabletten und Kapseln in Frage, die den Wirkstoff direkt mit sofortiger Freigabe oder kontrolliert freisetzen. Die Kapselhüllen bestehen dabei vorzugsweise aus einer Basis von Cellulose, Hydroxypropylcellulose oder Stärke, und als Tabletten kommen sowohl monolithische als auch multipartikuläre Formen in Frage. Erfindungsgemäß können die Tabletten durch die aus dem Stand der Technik bekannten Verfahren wie Direkttablettierung, Feuchtgranulation mit anschließendem Verpressen oder Trockengranulation mit anschließendem Verpressen erhalten und die so erhaltenen Arzneimittelfonnen z.B. wachspoliert oder beschichtet werden. Zur Gewährleistung einer verzögerten oder kontrollierten Freisetzung des Wirkstoffs kommen ebenfalls alle aus dem Stand der Technik bekannten Verfahren, wie Matrix- und diffusionskontrollierte membranbeschichtete Formulierungen, in Frage.

Die so erhaltenen, erfindungsgemäßen Arzneimittel zeigen völlig überraschend ohne Zusatz eines basischen Hilfsstoffes eine außerordentliche Lagerstabilität, wie durch Vergleichsmessungen mit durch den Zusatz alkalisierender Mittel stabilisierten Produkten unter Stressbedingungen bestätigt wird. Dabei werden die herausragenden Stabilitäten der erfindungsgemäßen Zubereitungen bei Formulierungen beobachtet, die in wässriger Lösung bzw. wässriger Dispersion pH-Werte unterhalb 8 ergeben; bevorzugt unterhalb 7.

In den folgenden Beispielen werden die erfindungsgemäßen Zusammensetzungen nach den angegebenen Verfahren hergestellt, die in den Rezepturen angegebenen Hilfs- bzw. Wirkstoffmengen in 10 ml bzw. 100 ml Wasser gelöst bzw. dispergiert und die pH-Werte mittels einer pH-Elektrode gemessen. Die Beispiele sollen die Erfindung näher erläutern, sie aber nicht einschränken.

### Beispiel 1

| **Rezeptur Fluvastatin 20mg Kapsel:** | |
|---|---|
| *Bestandteil* | *mg*/*Kapsel* |
| Fluvastatin Natrium | 21.06 |
| Lactose Monohydrat | 89.64 |
| Polyvinylpyrrolidon | 3.37 |
| Polyvinylpyrrolidon quervernetzt | 3.37 |
| Siliciumdioxid ungepr. | 0.34 |
| Magnesiumstearat | 1.02 |
| Talcum | 1.20 |
| **Füllgewicht Kapsel:** | 120.00 |

### Herstellungsprozess:

1. Fluvastatin Natrium, Lactose monohydrat, Polyvinylpyrrolidon, Polyvinylpyrrolidon quervernetzt, Siliciumdioxid ungepr. und Magnesiumstearat werden gemischt und zu Tabletten verpresst. Danach werden die Tabletten mittels eines Siebes zu einem Granulat verarbeitet.
2. Lactose monohydrat, Polyvinylpyrrolidon, Polyvinylpyrrolidon quervernetzt und Talcum werden zum Wirkstoffgranulat hinzugemischt.
3. Die Endmischung wird in Kapseln gefüllt (Gelatine oder Cellulose Kapseln)

Die pH-Werte des Wirkstoffs ohne Hilfsstoffe, der Hilfsstoffe ohne Wirkstoff sowie des fertigen Arzneimittels dieser Rezeptur wurden wie folgt bestimmt:

| pH-Werte der **Fluvastatin 20mg Kapsel-Formulierung:** | | |
|---|---|---|
| | pH-Wert in 10ml Wasser | pH-Wert in 100ml Wasser |
| Wirkstoff ohne Hilfsstoffe | 7.08 | 6.93 |
| Hilfsstoffe ohne Kapsel | 6.79 | 6.93 |
| Hilfsstoffe mit Gelantinekapsel | 5.42 | 5.56 |
| Hilfsstoffe mit Cellulosekapsel | 6.59 | 7.08 |
| Arzneimittel ohne Kapsel | 7.34 | 7.22 |
| Arzneimittel mit Gelantinekapsel | 6.71 | 6.43 |
| Arzneimittel mit Cellulosekapsel | 7.41 | 7.12 |

### Beispiel 2

| **Rezeptur Fluvastatin 80mg Retardtablette:** | |
|---|---|
| *Bestandteil* | *mg*/*Kapsel* |
| Fluvastatin Natrium | 84.24 |
| Lactose Monohydrat | 111.94 |
| Polyvinylpyrrolidon | 4.21 |
| Polyvinylpyrrolidon quervernetzt | 4.21 |
| mikrofeine Cellulose | 45.50 |
| Carrageen | 164.00 |
| Siliciumdioxid ungepr. | 4.15 |
| Magnesiumstearat | 3.30 |
| **Endgewicht Tablettenkern:** | 421.55 |
| | |

| Filmüberzug: | |
|---|---|
| Opadry® white** | 13.45 |
| Wasser dem.* | 80.00 |
| **Endgewicht Filmtablette:** | 435.00 |

| | |
|---|---|
| * Im Endprodukt nicht mehr enthalten ** Opadry® white: Hydroxypropylmethylcellulose, PEG, Titandioxid | |

### Herstellungsprozess:

1. Fluvastatin Natrium, Lactose monohydrat, Polyvinylpyrrolidon, Polyvinylpyrrolidon quervernetzt, Siliciumdioxid ungepr. und Magnesiumstearat werden gemischt und zu Tabletten verpresst. Danach werden die Tabletten mittels eines Siebes zu einem Granulat verarbeitet.
2. Lactose monohydrat, Polyvinylpyrrolidon, Polyvinylpyrrolidon quervernetzt, mikrofeine Cellulose, Carrageen, Siliciumdioxid ungepr. und Magensiumstearat werden zum Wirkstoffgranulat hinzugemischt und die Endmischung zu Tabletten abgepresst.
3. Die Tabletten werden zu Filmtabletten weiterverarbeitet.

Die pH-Werte des Wirkstoffs ohne Hilfsstoffe, der Hilfsstoffe ohne Wirkstoff sowie des fertigen Arzneimittels dieser Rezeptur wurden wie folgt bestimmt:

| pH-Werte der **Fluvastatin 80mg Retard-Tablettenformulierung:** | | |
|---|---|---|
| | pH-Wert in 10ml Wasser | pH-Wert in 100m1 Wasser |
| Wirkstoff ohne Hilfsstoffe | 7.08 | 6.93 |
| Hilfsstoffe | 7.69 | 7.32 |
| Arzneimittel (zermörsert) | 7.01 | 7.22 |

### Beispiel 3:

| **Rezeptur Fluvastatin 20mg Kapsel:** | |
|---|---|
| *Bestandteil* | *mg*/*Kapsel* |
| Fluvastatin Natrium | 21.06 |
| Lactose Monohydrat | 83.28 |
| Polyvinylpyrrolidon | 3.37 |
| Polyvinylpyrrolidon quervernetzt | 3.37 |
| Celluloseether | 6.36 |
| Talcum | 2.56 |
| **Füllgewicht Kapsel:** | 120.00 |

### Herstellungsprozess:

1. Fluvastatin Natrium, Lactose monohydrat, Polyvinylpyrrolidon, Polyvinylpyrrolidon quervernetzt, Celluloseether und Talcum werden gemischt.
2. Die Endmischung wird in Kapseln gefüllt (Gelatine oder Cellulose Kapseln)

### Beispiel 4:

| **Rezeptur Fluvastatin 20mg Kapsel:** | |
|---|---|
| *Bestandteil* | *mg*/*Kapsel* |
| Fluvastatin Natrium | 21.06 |
| Mikrokristalline Cellulose | 90.00 |
| Polyvinylpyrrolidon | 7.74 |
| Talcum | 1.20 |
| Wasser dem. | q.s. |
| **Füllgewicht Kapsel:** | 120.00 |

### Herstellungsprozess:

1. Fluvastatin Natrium und mikrokristalline Cellulose werden gemischt.
2. Polyvinylpyrrolidon wird in Wasser gelöst und die Mischung aus 1. granuliert.
3. Die Mischung wird getrocknet und mit Talcum versetzt.
4. Die Endmischung wird in Kapseln gefüllt (Gelatine oder Cellulose Kapseln).

### Beispiel 5:

| **Rezeptur Fluvastatin 80mg Retardtablette:** | |
|---|---|
| *Bestandteil* | *mg*/*Kapsel* |
| Fluvastatin Natrium | 84.24 |
| Mikrokrisalline Cellulose | 158.04 |
| Polyvinylpyrrolidon | 4.21 |
| mikrofeine Cellulose | 167.61 |
| Siliciumdioxid ungepr | 4.15 |
| Magnesiumstearat | 3.30 |
| **Endgewicht Tablettenkern:** | 421.55 |
| | |

| Filmüberzug: | |
|---|---|
| Opadry® white** | 13.45 |
| Wasser dem.* | 80.00 |
| **Endgewicht Filmtablette:** | 435.00 |

| | |
|---|---|
| * Im Endprodukt nicht mehr enthalten ** Opadry® white: Hydroxypropylmethylcellulose, PEG, Titandioxid | |

### Herstellungsprozess:

1. Fluvastatin Natrium, mikrokristalline Cellulose und mikrofeine Cellulose werden gemischt.
2. Polyvinylpyrrolidon wird in Wasser gelöst und die Mischung aus 1. granuliert.
3. Die Mischung wird getrocknet und mit Siliciumdioxid ungepr. und Magnesiumstearat versetzt und die Mischung zu Tabletten verpresst.
4. Die Tabletten werden zu Filmtabletten weiterverarbeitet.

### Beispiel 6:

| **Rezeptur Fluvastatin 80mg Retardtablette:** | |
|---|---|
| *Bestandteil* | *mg*/*Kapsel* |
| Fluvastatin Natrium | 84.24 |
| Lactose Monohydrat | 134.36 |
| Mikrofeine Cellulose | 45.50 |
| Celluloseether | 150.00 |
| Siliciumdioxid ungepr. | 4.15 |
| Magnesiumstearat | 3.30 |
| **Endgewicht Tablettenkern:** | 421.55 |
| | |

| Filmüberzug: | |
|---|---|
| Opadry® white** | 13.45 |
| Wasser dem.* | 80.00 |
| **Endgewicht Filmtablette:** | 435.00 |

| | |
|---|---|
| * Im Endprodukt nicht mehr enthalten ** Opadry® white: Hydroxypropylmethylcellulose, PEG, Titandioxid | |

### Herstellungsprozess:

1. Fluvastatin Natrium, Lactose monohydrat, mikrofeine Cellulose, Celluloseether, Siliciumdioxid ungepr. und Magnesiumstearat werden gemischt und zu Tabletten verpresst.
2. Die Tabletten werden zu Filmtabletten weiterverarbeitet.

### Beispiel 7:

Die Stabilitäten der erfindungsgemäßen Formulierungen wurden anhand der Bildungen von Verunreinigungen bzw. Umlagerungs- und Zersetzungsprodukten des Fluvastatins unter Stressbedingungen ermittelt und mit denen des kommerziell erhältlichen Produktes "Locol^{®}", das als Wirkstoff Fluvastatin Natrium und als basische Hilfsstoffe Calciumcarbonat und Natriumhydrogencarbonat enthält, verglichen. Die dabei durch Anwendung von HPLC-Methoden *(High Performance Liquid Chromatography)* erhaltenen Werte sind in der folgenden Tabelle zusammengestellt.

| | | |
|---|---|---|
| Stabilitätsuntersuchungen von Fluvastatin-Natrium enthaltenden Zubereitungen* | | |
| | Startwert | Werte nach 3 Tagen |
| Vergleichsprodukt | Größte Verunreinigung: 0.25% | Größte Verunreinigung: 1.85% |
| | Gesamtverunreinigung: 0.25% | Gesamtverunreinigung: 2.63% |
| Erfindungsgemäße | Größte Verunreinigung: 0.45% | Größte Verunreinigung: 1.34% |
| Zubereitung | Gesamtverunreinigung: 0.45% | Gesamtverunreinigung: 1.34% |
| (entsprechend Beispiel 1) | | |

| | | |
|---|---|---|
| *) offen, 60°C, 75% r.H. | | |

## Patentansprüche

1. Orales, lagerstabiles Arzneimittel umfassend einen HMG-CoA-Reduktase-Inhibitor der Formel Iₐ oder I_{b} 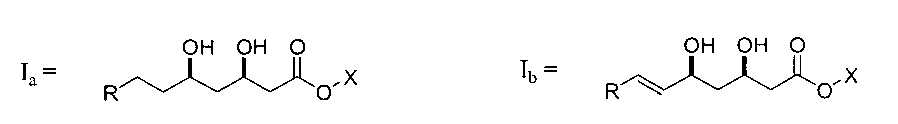 umfassen, worin R jeder organische Rest sein kann aber vorzugsweise aus 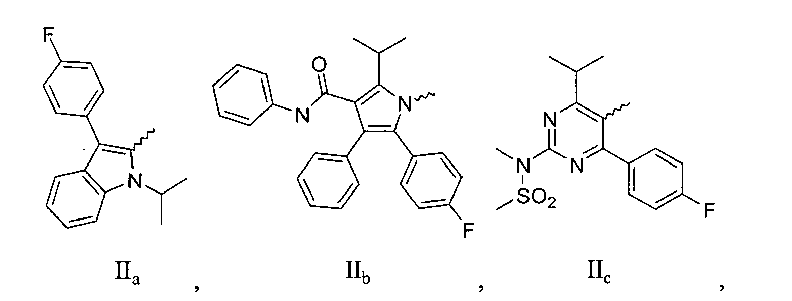 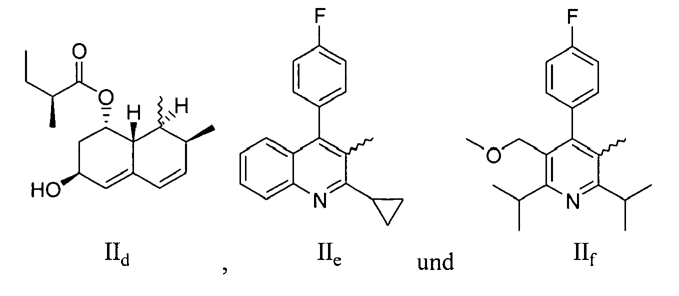 ausgewählt ist, und X aus der Gruppe Wasserstoff und Metall ausgewählt ist, und wenigstens einen pharmazeutischen Hilfsstoff, wobei die pharmazeutischen Hilfsstoffe nicht alkalisierend wirken.

2. Pharmazeutisches Mittel gemäß Anspruch 1, dessen wässerige Dispersion oder Lösung einen pH-Wert unterhalb pH = 8 aufweist.

3. Pharmazeutisches Mittel gemäß Anspruch 1 oder 2, dessen wässerige Dispersion oder Lösung einen pH-Wert unterhalb pH = 7 aufweist.

4. Pharmazeutisches Mittel nach einem der Ansprüche 1 bis 3, wobei der HMG-CoA-Reduktase-Inhibitor aus der Gruppe Fluvastatin, Rosuvastatin, Atorvastatin und Pravastatin sowie deren Salze ausgewählt ist.

5. Pharmazeutisches Mittel nach einem der Ansprüche 1 bis 4, wobei der HMG-CoA-Reduktase-Inhibitor Fluvastatin Natrium ist.

6. Pharmazeutisches Mittel nach einem der Ansprüche 1 bis 4, wobei der HMG-CoA-Reduktase-Inhibitor Rosuvastatin Calcium ist.

7. Pharmazeutisches Mittel nach einem der Ansprüche 1 bis 6, wobei der HMG-CoA-Reduktase-Inhibitor Atorvastatin Calcium ist.

8. Pharmazeutisches Mittel nach einem der Ansprüche 1 bis 7, wobei der pharmazeutische Hilfsstoff ausgewählt ist aus der Gruppe mikrokristalline oder mikrofeine Cellulose, Celluloseether, Lactose, mehrwertige Zucker, Polyacrylderivate, Carrageene, Polyvinylpyrrolidon, Polyvinylpyrrolidon quervernetzt, Polyvinylalkohol, Polyvinylacetat, Magnesiumstearat, hochdisperse Kieselsäure und Talcum.

9. Pharmazeutisches Mittel gemäß einem der Ansprüche 1 bis 8 in Form eines festen, einzeldosierten Arzneimittels.

10. Pharmazeutisches Mittel gemäß einem der Ansprüche 1 bis 9 mit direkter Freisetzung.

11. Pharmazeutisches Mittel gemäß einem der Ansprüche 1 bis 9 mit kontrollierter oder verzögerter Freisetzung.
